# EUROPEAN PATENT APPLICATION

(11) **EP 1 459 672 A2**
(43) Date of publication of application: **22.09.2004**
(21) Application number: 04251489.3
(22) Date of filing: 16.03.2004
(51) Int. Cl.: A47K 7/00

(54) **Expandable skin cleansing implement**

(30) Priority: 17.03.2003 US 390095
(71) Applicant: Johnson & Johnson Consumer Companies, Inc., Skillman, New Jersey 08558-9418 (US)
(72) Inventor: Lambino, Danilo L., Kogarah NSW 2113 (AU); Dabi, Shmuel, Highland Park, NJ 08904 (US); Siegwart, Kathleen, Milford, NJ 08848 (US); Edwards, Elizabeth Pletcher, Lawrenceville, NJ 08648 (US)
(74) Representative: Mercer, Christopher Paul

(57) **Abstract**

A cleansing implement including: a first outer layer; a second outer layer; and an inner layer disposed and retained between the outer layers, wherein at least one of the outer layers is water permeable, and the inner layer includes means for expanding the implement to at least about 1.5 times its original height is disclosed.

## Description

### Background Of The Invention

The present invention relates to an improved skin cleansing implement. The skin cleansing implement is expandable. The expandable skin cleansing implement is convenient for storage at home and in luggage while travelling.

Many consumers achieve personal cleansing by using a bar of soap as a cleansing material and an implement, such as a washcloth, a sponge, or the like to apply the soap to the body. Recently, liquid cleansers have become increasingly more prevalent. The liquid cleanser is typically applied to the body with an implement, such as a washcloth, a sponge, a puff/pouf and the like. Gathered implements, such as puffs/poufs have gained in popularity due to their ability to provide cleansing and exfoliation benefits.

One problem associated with cleansing implements is that they tend to be relatively large. For example, typical pouf implements have a diameter ranging from about 3 inches to about 6 inches. Solid cleanser holder implements are also known. The implements are typically in the shape of a conventional soap bar. These implements are approximately 3 inches long, 3 inches wide, and 1 inch thick. These cleansing implements take up significant space, both on a shelf and in luggage. There is a need for a cleansing implement that is relatively thin prior to use, and expands during use.

Commercially available cleansing implements are sometimes perceived by consumers as being too hard or too soft on the skin. There is a need for a cleansing implement that feels just right on the skin.

United States Patent No. 5,727,278 describes a cleansing device that is a reticulated polyurethane sponge. The reticulated polyurethane sponge does not expand during use.

United States Patent Nos 6,092,257 and 6,038,727 describe cleansing devices. The devices have various textures and fibers to produce a multi-layer ruffled body forming a "bath ball".

United States Patent 6,015,242 describes a body cleansing puff which contains pieces of solid soap. The device is filled with solid soap and re-filled as the solid soap is used.

United States Patent 6,368,003 describes a hand-held washing device that contains soap within the interior. The soap is contained within a reservoir and dispensed via a nozzle. The soap may be in a variety of forms including bar and fluid.

European Patent Application No. EP 1125540 describes a textured film cleansing device made from at least one layer of gathered textured film.

United States Patent 6,063,397 describes dry wipes that are useful for personal cleansing.

Despite the disclosure of the references, there is a continuing need for a cleansing implement that is relatively thin prior to use, and expands during use. The present invention answers this need.

### Summary Of The Invention

The present invention provides a cleansing implement including a first outer layer; a second outer layer; and an inner layer disposed and retained between the outer layers, wherein at least one of said outer layers is water permeable and the inner layer expands upon contact with water to expand the cleansing implement.

### Detailed Description Of The Invention

The cleansing implement of the present invention has a first outer layer and a second outer layer. The first outer layer and the second outer layer may be made of the same material, or may be made of different materials. At least one of the first outer layer and second outer layer must be made of a water permeable material. Suitable materials for the first and second outer layers are known in the art of wipes and include, but are not limited to, a woven fabric, a knit fabric, a nonwoven fabric, a laminate of a fabric and a polymeric film, such as a polyolefin film, a flocked fabric, a polyolefin film, apertured films, open-mesh netting, and combinations thereof. Suitable polymeric film materials include, but are not limited to, polyolefins, such as polyethylene, low density polyethylene, linear low density polyethylene, high density polyethylene, and polypropylene, polyesters, and ethylene vinyl acetate.

Methods of making woven and knit cloths are not a part of this invention and, being well known in the art, are not described in detail herein. One type of nonwoven cloth substrate utilized in the present invention is made by air- or water-laying processes in which the fibers or filaments are first cut to desired lengths from long strands, passed into a water or air stream, and then deposited onto a screen through which the fiber-laden air or water is passed. The deposited fibers or filaments are then adhesively bonded together, and otherwise treated as desired to form the woven, nonwoven, or cellulose cloth.

The first and second outer layers utilized in the present invention may be a thermal bonded nonwoven cloth (whether or not resin-containing) which can be made of polyesters, polyamides, polyolefins, or other thermoplastic fibers which can be spun bonded, i.e., the fibers are spun out onto a flat surface and bonded (melted) together by heat or chemical reactions.

When nonwovens are utilized, the nonwoven cloth substrates are generally adhesively bonded fibers or filamentous products having a web or carded fiber structure (when the fiber strength is suitable to allow carding) or comprising fibrous mats in which the fibers or filaments are distributed haphazardly or in random array (i.e., an array of fibers in a carded web where partial orientation of the fibers is frequently present, as well as a completely haphazard distributional orientation), or substantially aligned. The fibers or filaments can be natural (e.g., wool, silk, jute, hemp, cotton, linen, sisal, or ramie) or synthetic (e.g., rayon, cellulose ester, polyvinyl derivatives, polyolefins, such as polyethylene and polypropylene, polyamides, such as nylon 6, nylon 6,6, or polyesters, such as polyethylene terephthalate and polybutylene terephthalate), or combinations thereof. These nonwoven materials are generally described in the INDA "NONWOVEN FABRICS HANDBOOK", (1999) for nonwoven substrates and their methods of manufacture.

The basis weight of the nonwoven outer layers may vary, but generally ranges from about 20 grams per square meter to about 500 grams per square meter, for example from about 50 grams per square meter to about 150 grams per square meter.

The inner layer is retained between the first outer layer and the second outer layer. Suitable mechanism for retaining the inner layer are known in the art, and include, but are not limited to, adhesive bonding, thermal bonding, sewing, hooks and loops, and the like. Generally, the first and second outer layers may be sealed together on the perimeter by the above-mentioned mechanisms.

The implement of the present invention has an inner layer disposed between the outer layers. The inner layer expands upon contact with water thereby expanding the implement. As used herein, expanding the implement means increasing the height of the implement. During storage, the implement has an initial height. Upon contact with water, the height of the implement increases to a final height. The final height of the implement is greater than about 1.5 times the initial height of the implement, and ranges from about 1.5 to about 10, preferably from about 3 to about 5 times the initial height of the implement.

The inner layer may be any material that will expand upon contact with water. Examples of suitable materials include absorbent powders, such as superabsorbent polyacrylic acid salts and the like. Superabsorbent fibers and superabsorbent mats may also be useful. One useful superabsorbent mat is a mixture of wood cellulose and a polyacrylate, available as NOVATHIN™ from EAM Corporation. Another useful superabsorbent material is an inorganic salt of olefin/alkyl carboxylate copolymer available as FIBERDRI™ fiber from Camelot Technologies Ltd.

Suitable absorbent powders further include, but are not limited to, natural polymers, such as karaya gum, tragacanth gum, gum Arabic, gum Ghatti, guar gum, locust bean gum, quince seed, psyllium seed, carageenan, alginates, agar, pectins, starches, Xanthan gum, Dextran, casein, gelatin, keratin, and shellac; modified natural polymers, such as cellulose derivatives including hydroxypropyl cellulose and hydroxyethyl cellulose, and hydroxypropyl guar; synthetic polymers such as acrylic acid polymers, polyacrylamides, and alkylene/alkylene oxide polymers; and inorganics such as clays and amorphous silicon dioxide.

Compressed materials, such as sponges, nonwovens, and apertured films may also be used as the inner layer of the implement. The sponges may also be made of cellulose derivatives such as viscose, cellulose ester and cellulose ether, and combinations, thereof. Suitable nonwoven materials include those described above. High loft nonwovens are particularly useful. In order to obtain this expandable core, a sponge or nonwoven material is compressed, for example in a press. Heat may be applied to the sponge or nonwoven material while it is being compressed. Water soluble binders may be applied to the sponge or nonwoven material to keep the material compressed until the implement is contacted with water. Suitable water soluble binders include, but are not limited to, starch based binders, acrylate based binders, and the like. Upon contact with water, the water soluble binder dissolves and the inner layer (in this case the sponge or nonwoven) expands.

The inner layer may be impregnated with a cleansing composition by means known in the art. Suitable cleansing compositions generally include one or more surfactants. Typically, a lathering surfactant is included in the cleansing composition. What is meant by a lathering surfactant is a surfactant that generates lather when combined with water and mechanically agitated. Examples of lathering surfactants include, but are not limited to, anionic, nonionic, cationic, and amphoteric lathering surfactants.

Nonlimiting examples of anionic lathering surfactants include those selected from the group consisting of sarcosinates, sulfates, isethionates, taurates, phosphates, lactylates, and glutamates. Specific examples include, but are not limited to, those selected from the group consisting of sodium lauryl sulfate, ammonium lauryl sulfate, ammonium laureth sulfate, sodium laureth sulfate, sodium trideceth sulfate, ammonium cetyl sulfate, sodium cetyl sulfate, ammonium cocoyl isethionate, sodium lauroyl isethionate, sodium lauroyl lactylate, triethanolamine lauroyl lactylate, sodium caproyl lactylate, sodium lauroyl sarcosinate, sodium myristoyl sarcosinate, sodium cocoyl sarcosinate, sodium lauroyl methyl taurate, sodium cocoyl methyl taurate, sodium lauroyl glutamate, sodium myristoyl glutamate, and sodium cocoyl glutamate and mixtures thereof.

Nonlimiting examples of nonionic lathering surfactants include those selected from the group consisting of alkyl glucosides, alkyl polyglucosides, polyhydroxy fatty acid amides, alkoxylated fatty acid esters, lathering sucrose esters, amine oxides, and mixtures thereof. Specific examples include, but are not limited to, nonionic surfactants to those selected form the group consisting of C8-C 14 glucose amides, C8- C14 alkyl polyglucosides, sucrose cocoate, sucrose laurate, lauramine oxide, cocoamine oxide, and mixtures thereof.

Nonlimiting examples of amphoteric lathering surfactants (which also includes zwitterionic lathering surfactants) are those selected from the group consisting of betaines, sultaines, hydroxysultaines, alkyliminoacetates, iminodialkanoates, aminoalkanoates, and mixtures thereof.

Nonlimiting examples of amphoteric surfactants of the present invention include disodium lauroamphodiacetate, sodium lauroamphoacetate, cetyl dimethyl betaine, cocoamidopropyl betaine, cocoamidopropyl hydroxy sultaine, and mixtures thereof.

Skin care actives, moisturizers, and the like may also be impregnated into the implements of the present invention. The compositions may be loaded onto the inner layer by dipping the implement in the composition, spraying the composition onto the implement, and other means known in the art. The inner layer may be dried through the use of heating equipment or vacuum driers to provide dry implements. Alternatively, a cleansing or skin care formulation may be applied in the form of a concentrate to the inner layer to provide dry implements.

The cleansing implements are sold dry. The consumer wets the implement with water when ready for use. As used herein, "dry" means that the cleansing implement contains less than about 15 percent by weight, preferably less than about 10 percent by weight water, based on the total weight of the cleansing implement. Upon contact with water, the implement expands.

The present invention also provides skin cleansing implements that do not feel too hard or too soft on the skin. These implements have wet compression in the Z direction scores ranging from about 100 to about 5,000. Preferably the wet compression scores range from about 500 to about 3,000. Wet compression in the Y direction scores may range from about 100 to about 5,000. Preferably the wet compression in the Y direction scores range from about 500 to about 3,000. "Wet compression" is described in detail below in the Examples.

The following examples are provided for illustrative purposes. The invention should not be construed as being limited to the details thereof.

### EXAMPLES

The following test methods were employed in the Examples.

### Dry Compression/Resilience Test Method

This test method uses an Instron Model II22 to measure the peak load of a sample at a given height of compression as well as the height that the sample returns to when the load returns to zero load. All tests were conducted at 23° C and 50% relative humidity. A 50 1b load/compression cell was mounted onto the machine. The load cell was plugged in and allowed to warm up for at least 20 minutes. The machine was then calibrated with a 5 1b weight. A custom-made probe was used in this particular test because it is most representative of a hand squeezing the implement.

The caliper of the sample was measured in the direction in which it is to be compressed (either the Y or Z direction). This measurement was divided in half and set as the elongation point for the sample. The sample was centered under the probe with the sample's longest edge parallel to the probe's longest edge. For compression in the Y direction, two metal pieces (approximately 25 mm x 40 mm x 25 mm) were affixed to the bottom plate, with the width between them approximately the width of the sample in the Z direction, and the 40 mm edge of the metal piece in contact with the plate. The sample was placed between the metal pieces for testing so that it did not move between cycles. Furthermore, the "fringe" (excess material beyond the heat seal) of the samples tested in the Y direction was cut off to the outer edge of the heat seal to avoid unrealistic data that may be caused by it.

Once the sample was centered, the probe was brought down until it just slightly touched the sample (the load is less than 1 g-force) and the load was set to zero. After this was done and the elongation point had been set, the test was ready to begin.

The probe was extended into the sample until it reached the elongation point and then retrieved back, all at a speed of 12 inches/minute. This cycle was repeated nine times, for a total of ten cycles. Afterward, the peak load (at the elongation point) was reported for each sample. In addition, for each cycle, the height on the return of the probe at which the load returned to zero was recorded. This was considered the "return height" of the sample. The return height divided by the original height is distinguished as "% Resiliency".

### Wet Compression/Resilience Test Method

The Wet Compression/Resilience Test Method followed the same procedure as the Dry Compression/Resilience Test Method up to and including the point where the caliper of the sample was measured. A plastic tray was placed on the bottom plate of the Instron to catch excess water (in the case of compression in the Y direction, the metal pieces were affixed to this plastic tray rather than the bottom plate). Next, the sample was submersed in water for 10 seconds, all the while being manually turned over in the water approximately once per second. The sample was removed with the XY plane parallel to the surface of the water. The sample was centered and the zeroing of the load and cycle test proceeded in the same manner as in the Dry Compression/Resilience Test Method. Again, the peak load and return height were recorded for each cycle.

### Wet Compression/Resilience Test Method for Water Activated Expandable Implement

In the case of an implement that begins in a relatively flat state and is water-activated to expand, only the Wet Compression/Resilience Test Method was applied. In addition, two modifications were made: the sample was submersed for a certain period of time without being turned over once per second (see individual examples for further details) and the caliper was not measured until after the water submersion had taken place. The rest of the test followed the Wet Compression/Resilience Test Method.

### Water-activated Expansion Test

The cleansing implements expand into a soft cleansing pouch upon contact with sufficient amount of water. The water-activated expansion is measured directly in terms of % change in thickness and weight of the cleansing implement after immersion in 1 liter of water from 0 to 120 seconds, at 30-second intervals.

### Example 1: Samples 1 - 3 - Implements Containing Polyester High Loft

Sample 1 was made of two outer layers of non-woven material and an inner core of high loft nonwoven material. Each outer layer was a 89 mm x 127 mm polypropylene spunbond nonwoven material from PGI. The nonwoven had a basis weight of 40 to 45 grams per square meter ("gsm"). The inner core was a 102 mm x 610 mm high loft polyester from Union Wadding. The basis weight was 102 gsm and the relaxed caliper was 3.5.

The two outer layers were layered together and heat-sealed on three sides to form a pouch, with the fringe on the outside of the seal not exceeding 3 mm. The high loft was rolled up in the direction of the length of the piece until the whole piece was in a cylinder like-shape. The roll was flattened slightly to insert into the pouch. The last edge of the outer layers was then heat-sealed.

Sample 2 was made of two outer layers of material and an inner core of high loft nonwoven material. Each outer layer was a 89 mm x 127 mm polypropylene spunbond nonwoven material from PGI. The nonwoven had a basis weight of 40 to 45 gsm. The inner core was a 102 mm x 305 mm high loft polyester from Union Wadding. The basis weight was 102 gsm and the relaxed caliper was 3.5.

The two outer layers were layered together and heat-sealed on three sides to form a pouch, with the fringe on the outside of the seal not exceeding 3 mm. The high loft was rolled up in the direction of the length of the piece until the whole piece was in a cylinder like-shape. The roll was flattened slightly to insert into the pouch. The last edge of the outer layers was then heat-sealed.

Sample 3 was made of two outer layers of material and an inner core of high loft nonwoven material. Each outer layer was a 89 mm x 127 mm polypropylene spunbond nonwoven material from PGI. The nonwoven had a basis weight of 40 to 45 gsm. The inner core was a 102 mm x 140 mm high loft polyester from Union Wadding. The basis weight was 102 gsm and the relaxed caliper was 3.5.

The two outer layers were layered together and heat-sealed on three sides to form a pouch, with the fringe on the outside of the seal not exceeding 3 mm. The high loft was folded in half in the direction of the length of the piece and inserted into the pouch. The last edge of the outer layers was then heat-sealed.

### Example 2: Samples 4 - 6 - Implements Containing Apertured Film

Sample 4 consisted of two outer layers of material and an inner core of apertured film. Each outer layer was a 89 mm x 127 mm polypropylene spunbond nonwoven material from PGI. The nonwoven had a basis weight of 40 to 45 gsm. The inner core for this pouch was an ethylene vinyl acetate copolymer blend hexagonal apertured film from Tredegar Film Products. The basis weight of the film was 30 gsm. Eight 120 mm x 610 mm pieces of film were used to make the inner core for this sample.

The two outer layers were layered together and heat-sealed on three sides to form a pouch, with the fringe on the outside of the seal not exceeding 3 mm. The pieces of film were layered in the following manner: male side up, then male side down, male side up, then male side down, and so on. Once the 8 pieces of film were layered, they were folded. The layers were folded inward at the ¼ and ¾ mark of the length of the pieces, to meet the ½ mark. The film was then folded in half. Finally, the film was folded in thirds. In this configuration, the film was stuffed into the pouch and the open side of the pouch was heat sealed, with the fringe being no more than 3 mm.

Sample 5 consisted of two outer layers of material and an inner core of apertured film. Each outer layer was a 89 mm x 127 mm polypropylene spunbond nonwoven material from PGI. The nonwoven had a basis weight of 40 to 45 gsm. The inner core for this pouch was an ethylene vinyl acetate copolymer blend hexagonal apertured film from Tredegar Film Products. The basis weight of the film was 30 gsm. Four 102 mm x 610 mm pieces of film were used to make the inner core for this sample.

The two outer layers were layered together and heat-sealed on three sides to form a pouch, with the fringe on the outside of the seal not exceeding 3 mm. The pieces of film were layered in the following manner: male side up, then male side down, male side up, then male side down. Once the 4 pieces of film were layered, they were folded. The layers were folded inward at the ¼ and ¾ mark of the length of the pieces, to meet the ½ mark. The film was then folded in half. Finally, the film was folded in thirds. In this configuration, the film was stuffed into the pouch and the open side of the pouch was heat sealed, with the fringe being no more than 3 mm.

Sample 6 consisted of two outer layers of material and an inner core of apertured film. Each outer layer was a 89 mm x 127 mm polypropylene spunbond nonwoven material from PGI. The nonwoven had a basis weight of 40 to 45 gsm. The inner core for this pouch was an ethylene vinyl acetate copolymer blend hexagonal apertured film from Tredegar Film Products. The basis weight of the film was 30 gsm. Two 102 mm x 610 mm pieces of film were used to make the inner core for this sample.

The two outer layers were layered together and heat-sealed on three sides to form a pouch, with the fringe on the outside of the seal not exceeding 3 mm. The pieces of film were layered in the following manner: male side up, then male side down. Once the 2 pieces of film were layered, they were folded. The layers were folded inward at the ¼ and ¾ mark of the length of the pieces, to meet the ½ mark. The film was then folded in half. Finally, the film was folded in thirds. In this configuration, the film was stuffed into the pouch and the open side of the pouch was heat sealed, with the fringe being no more than 3 mm.

### Example 3: Sample 7 - Implement Containing an Acrylic Bonded High Loft

Sample 7 consisted of two outer layers of material and an inner core of acrylic bonded high loft polyester. Each outer layer was a 89 mm x 127 mm polypropylene spunbond nonwoven material from PGI. The nonwoven had a basis weight of 40 to 45 gsm. The inner core was a 102 mm x 140 mm piece of acrylic bonded high loft polyester from Carlee Corporation. The basis weight of the material was 78 gsm and the relaxed caliper was 10.32.

The two outer layers were layered together and heat-sealed on three sides to form a pouch, with the fringe on the outside of the seal not exceeding 3 mm. The high loft was folded in half and inserted into the pouch. Finally, the last edge of the outer layers was heat-sealed.

### Example 4: Sample 8 - Implement Containing a Compressed Cellulose Material with a Skin Cleanser

Sample 8 consisted of two outer layers of material and an inner core of compressed cellulose sponge containing a mild surfactant system. Each outer layer was a 89 mm x 127 mm polypropylene spunbond nonwoven material from PGI. The nonwoven had a basis weight of 40 to 45 gsm. The inner core for this implement was a 50 mm x 90 mm compressed cellulose sponge that expands when wet. The basis weight of the sponge material was 623 gsm.

The two outer layers were layered together and heat-sealed on three sides to form a pouch, with the fringe on the outside of the seal not exceeding 3 mm. Approximately 2.0 grams of a commercially available skin cleanser was applied to the compressed sponge and allowed to air dry for 6 hours. The compressed sponge was finally inserted into the pouch and the last edge of the outer layers was heat-sealed. For testing according to the Wet Compression/Resilience Test Method for Water Activated Expandable Implement, this sample was submersed under water for 30 seconds before the wet caliper was measured.

### Example 5: Sample 9 - Implement Containing a Superabsorbent Pad with a Skin Cleanser

Sample 9 consisted of two outer layers of material and a superabsorbent pad containing a skin cleanser. Each outer layer was a 70 mm x 90 mm polyethylene copolymer blend pentagonal apertured film from Tredegar Film Products. The basis weight of the film was 30 gsm. The inner core for this implement was a 60 mm x 80 mm NOVATHIN™ superabsorbent pad from EAM Corporationi. The superabsorbent pad had a basis weight of 208 gsm.

The two outer layers were layered together and heat-sealed on three sides to form a pouch, with the fringe on the outside of the seal not exceeding 3 mm. Approximately 2.2 grams of a commercially available skin cleanser was applied to the pad and allowed to air dry for 6 hours. The pad was folded in half and inserted into the pouch. Finally, the last edge of the outer layers was heat-sealed. For testing according to the Wet Compression/Resilience Test Method for Water Activated Expandable Implement, this sample was submersed under water for 120 seconds before the wet caliper was measured.

### Example 6 - Dry and Wet Compression Results

The implements of samples 1-9 above were tested for dry compression, wet compression, and resiliencey. For dry compression and wet compression in the Z direction, the amount of force in grams to compress the implement to 50 percent of its initial height on the first cycle is reported in Table 1 below. The dry compression and wet compression in the Y direction data is reported in Table 2 below. For resiliency, the percent of initial height recovered after the tenth compression cycle is reported in Table 3.

**Table 1-**

| Z Direction Data | | |
|---|---|---|
| Sample | Wet Compression | Dry Compression |
| 1 | 2,317 | 2,516 |
| 2 | 955 | 1,338 |
| 3 | 847 | 162 |
| 4 | 1,683 | 1,811 |
| 5 | 549 | 588 |
| 6 | 150 | 166 |
| 7 | 1,417 | 134 |
| 8 | 1,156 | NA |
| 9 | 2,667 | NA |

| | | |
|---|---|---|
| NA = Not Analyzed | | |

**Table 2 -**

| Y Direction Data | | |
|---|---|---|
| Sample | Wet Compression | Dry Compression |
| 1 | 2602 | 2509 |
| 2 | 1245 | 1480 |
| 3 | 245 | 238 |
| 4 | 2353 | 2716 |
| 5 | 1168 | 1122 |
| 6 | 126 | 129 |
| 7 | 251 | 172 |
| 8 | 614 | NA |
| 9 | 304 | NA |

**Table 3-**

| Resiliency | |
|---|---|
| Sample | Resiliency |
| 1 | 81 |
| 2 | 77 |
| 3 | 72 |
| 4 | 84 |
| 5 | 87 |
| 6 | 80 |
| 7 | 72 |
| 8 | 69 |
| 9 | 60 |

### Example 7 - Water-Activated Expansion Results

The implements of samples 8 and 9 were tested for water-activated expansion. The percent increase in thickness of the implement after 60 seconds in water is reported in Table 4 below.

**Table 4**

| Sample | Expansion |
|---|---|
| 8 | 614 |
| 9 | 192 |

## Claims

1. A cleansing implement comprising:
a first outer layer;
a second outer layer; and
an inner layer disposed and retained between the outer layers,
wherein at least one of said outer layers is water permeable, and the inner layer expands upon contact with water to expand said implement to at least about 1.5 times its original height.

2. The cleansing implement according to claim 1 wherein the inner layer comprises an absorbent powder, a superabsorbent mat or a compressed material.

3. The cleansing implement according to claim 2 wherein the inner layer comprises an absorbent powder which is a natural polymer, a modified natural polymer, a synthetic polymer or an inorganic.

4. The cleansing implement according to claim 3 wherein the absorbent powder is a natural polymer which is karaya gum, tragacanth gum, gum Arabic, gum Ghatti, guar gum, locust bean gum, quince seed, psyllium seed, carageenan, an alginate, agar, a pectin, a starch, Xanthan gum, Dextran, casein, gelatin, keratin or shellac.

5. The cleansing implement according to claim 3 wherein the absorbent powder is a modified natural polymer which is hydroxypropyl cellulose, hydroxyethyl cellulose or hydroxypropyl guar.

6. The cleansing implement according to claim 3 wherein the absorbent powder is a synthetic polymer which is an acrylic acid polymer, a polyacrylamide or an alkylene/alkylene oxide polymer.

7. The cleansing implement according to claim 2 wherein the inner layer comprises a compressed material which is a sponge, nonwoven or apertured film.

8. The cleansing implement according to any one of claims 1 to 7 wherein, upon contact with water, the implement expands from 1.5 to 10 times its original height.

9. A cleansing implement comprising:
a first outer layer;
a second outer layer; and
an inner layer disposed and retained between the outer layers,
wherein at least one of said outer layers is water permeable, and the implement has a wet compression in the Z direction score ranging from 100 to 5,000.

10. A cleansing implement comprising:
a first outer layer;
a second outer layer; and
an inner layer disposed and retained between the outer layers,
wherein at least one of said outer layers is water permeable, and the implement has a wet compression in the Y direction score ranging from 100 to 5,000.
